(19) Europäisches Patentamt
European Patent Office
Office européen des brevets

(11) Veröffentlichungsnummer: **0 002 723**
**B1**

(12) # EUROPÄISCHE PATENTSCHRIFT

(21) Anmeldenummer: **78101656.3**

(22) Anmeldetag: **13.12.78**

(51) Int. Cl.³: **C 07 F 9/24, A 61 K 31/66**

(54) **Phosphonylureidobenzol-Derivate, Verfahren zu ihrer Herstellung und ihre Verwendung in Arzneimitteln**

(30) Priorität: **24.12.77 DE 2758005**

(43) Veröffentlichungstag der Anmeldung:
**11.07.79 Patentblatt 79/14**

(45) Bekanntmachung des Hinweises auf die Patenterteilung:
**21.01.81 Patentblatt 81/03**

(84) Benannte Vertragsstaaten:
**BE CH DE FR GB IT NL**

(56) Entgegenhaltungen:
**Keine**

(73) Patentinhaber: **BAYER AG**
**Zentralbereich Patente, Marken und Lizenzen**
**D - 5090 Leverkusen 1, Bayerwerk (DE)**

(72) Erfinder: **Wollweber, Hartmund, Dr.**
**In den Birken 73**
**D - 5600 Wuppertal 1 (DE)**
**Thomas, Herbert, Dr.**
**Bergerheide 82b**
**D - 5600 Wuppertal 1 (DE)**
**Andrews, Peter, Dr.**
**Am Eckbusch 35**
**D - 5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 723 B1

# Phosphonylureidobenzol-Derivate, Verfahren zu ihrer Herstellung und ihre Verwendung in Arzneimitteln

Die vorliegende Erfindung betrifft neue Phosphonylureido-benzol-Derivate, Verfahren zu ihrer Herstellung und ihre Verwendung in Arzneimitteln, insbesondere in Parasitiziden.

Aus verschiedenen Publikationen (z.B. DE—OS 2 029 298, DE—OS 2 029 299, US—PS 3 860 590) ist bereits bekannt geworden, daß N-[(substituierte)-Aminophenyl]-N',N'-dimethylacetamidine gegen Helminthen wirksam sind. Diese Verbindungen sind für die Behandlung von hauptsachlich bei Pflanzenfressern vorkommenden Helminthen gut geeignet, sind aber bei der Bekämpfung von Helminthen bei Carnivoren (Fleischfressern) weniger gut geeignet als die erfindungsgemäßen Wirkstoffe.

Die vorliegende Erfindung betrifft neue Phosphonylureido-benzol-Derivate der allgemeinen Formel

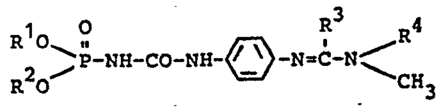

in welcher
$R^1$ und $R^2$ gleich oder verschieden sind und für Alkyl ($C_1$—$C_4$) stehen
$R^3$ und $R^4$ gleich oder verschieden sind und für Alkyl ($C_1$—$C_4$) stehen oder
$R^3$ und $R^4$ zusammen für eine Trimethylen- oder Tetramethylengruppe stehen oder
$R^3$ und $R^4$ zusammen mit dem durch sie eingeschlossenen Kohlenstoff- und Stickstoffatom ein Thiazolidin-Ringsystem bilden
sowie ihre physiologisch verträglichen Säureadditionssalze.

Als physiologisch verträgliche Säureadditions-Salze der erfindungsgemäßen Verbindungen seien beispielhaft genannt: Hydrochloride, Sulfate, Phosphate, Nitrate, Acetate, Methansulfonate, Naphthalindisulfonate, Pamoate, Fumarate, Maleinate.

Die erfindungsgemäßen Verbindungen der allgemeinen Formel (I) werden hergestellt, indem man Aminophenylamidine der allgemeinen Formel

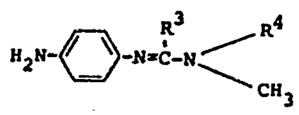

(II)

in welcher
$R^3$ und $R^4$ die oben angegebene Bedeutung besitzen,
oder deren Salze, mit Phosphonylisocyanaten der allgemeinen Formel

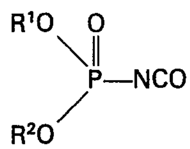

(III)

in welcher
$R^1$ und $R^2$ die oben angegebene Bedeutung besitzen,
gegebenenfalls in Anwesenheit eines Lösungsmittels umsetzt, das Reaktionsprodukt gegebenenfalls isoliert und gegebenenfalls durch Zusatz von Säure in physiologisch verträgliche Salze überführt.

Überraschenderweise zeigen die erfindungsgemäßen Phosphonylureidobenzol-Derivate gegenüber hauptsächlich bei Carnivoren vorkommenden Helminthen eine erheblich höhere Wirksamkeit als aus dem Stand der Technik bekannte Verbindungen. Die erfindungsgemäßen Stoffe stellen somit eine Bereicherung der Pharmazie dar.

Verwendet man Diäthylphosphonylisocyanat und N-(4-Aminophenyl)-N',N'-dimethylacetamidin als Ausgangsstoffe, so kann der Reaktionsablauf durch das folgende Formelschema wiedergegeben werden:

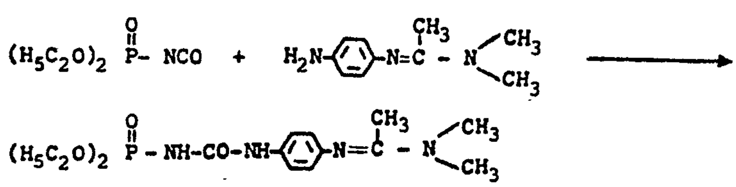

Die bei der Herstellung der erfindungsgemäßen Verbindungen einsetzbaren Aminophenylamidine der Formel (II) sind entweder bekannt oder können nach an sich bekannten Methoden hergestellt werden.

Als Herstellungsmethoden für die Ausgangsstoffe (II) seien z.B. aufgeführt:

a)

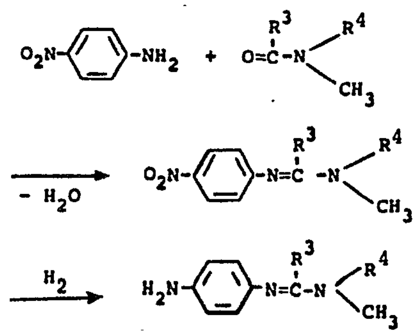

b)

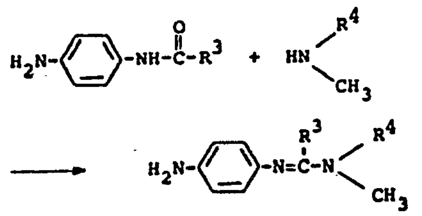

wobei die Substituenten $R^3$ und $R^4$ die oben angegebene Bedeutung besitzen. Siehe dazu auch DT—OS 2 029 298.

Beispielhaft seien als Aminophenylamidine der allgemeinen Formel (II) aufgeführt:

N-(4-Aminophenyl)-N',N'-dimethylacetamidin
N-(4-Aminophenyl)-N'-methyl-N'-äthylacetamidin
N-(4-Aminophenyl)-N',N'-diäthylacetamidin
N(N-Aminophenyl)-N',N'-dimethylpropionamidin
1-Methyl-2-(4-aminophenylamino)-pyrrolidin
1-Methyl-2-(4-aminophenylimino)-1,3-thiazolidin

Die Ausgangsprodukte der Formel (III) sind entweder bekannt oder können nach an sich bekannten Methoden, beispielsweise durch Umsetzung von Verbindungen der Formel

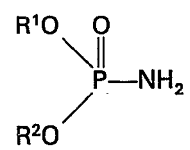

mit Oxalylchlorid leicht hergestellt werden, wobei $R^1$ und $R^2$ die oben angegebene Bedeutung besitzen:

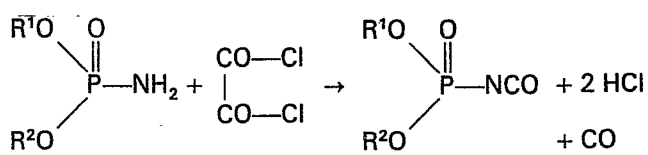

Als Phosphonylisocyanate der allgemeinen Formel (III) seien aufgeführt:

Dimethylphosphono-isocyanat
Dipropylphosphono-isocyanat
Äthyl-propylphosphono-isocyanat
Diisopropylphosphono-isocyanat
Dibutylphosphono-isocyanat

Die Umsetzung von Aminophenylamidinen der allgemeinen Formel (II) mit Phosphonylisocyanaten der allgemeinen Formel (III) kann mit bzw. ohne Verwendung eines Verdünnungsmittels vorgenommen werden, vorzugsweise wird jedoch in Gegenwart inerter Verdünnungsmittel gearbeitet. Geeignete Verdünnungsmittel sind beispielsweise aromatische Kohlenwasserstoffe wie Benzol, Toluol oder Xylol, chlorierte Kohlenwasserstoffe wie Chlorbenzol, Dichlorbenzole, Tetrachlorkohlenstoff, Chloroform, Methylenchlorid, Tetrachloräthylen, Trichloräthylen, Äther wie Dibutyläther, Tetrahydrofuran

oder polare Lösungsmittel wie Dimethylformamid, Dimethylsulfoxid, Alkohole wie Äthanol, Isopropanol, tert.-Butanol.

Die Umsetzung erfolgt bei Temperaturen von ca. 0 bis ca. 150°C, vorzugsweise bei Temperaturen von ca. 20 bis ca. 80°C. Im allgemeinen werden äquimolare Mengen an Komponente (II) und (III) eingesetzt, jedoch kann auch gegebenenfalls ein Überschuß an einer der beiden Komponenten (II) oder (III) eingesetzt werden.

Die Reaktionsprodukte des Formel (I) fallen entweder spontan oder nach dem Entfernen des Lösungsmittels, im allgemeinen kristallin, aus. Die Aufarbeitung erfolgt in an sich bekannter Weise durch Absaugen des Reaktionsprodukts und Umkristallisieren aus geeigneten Lösungsmitteln.

Die Reaktionsprodukte fallen, in Abhängigkeit von den eingesetzten Ausgangsverbindungen zum Teil als Säureadditionssalze an, diese können dann als solche gereinigt bzw. durch Zusatz von Säure in die zugrunde liegenden basischen Verbindungen der allgemeinen Formel (I) übergeführt werden.

Die Umsetzung von Verbindungen der Formel (II) mit Verbindungen der Formel (III) kann bei Normaldruck aber auch bei erhöhtem Druck durchgeführt werden. Vorzugsweise wird bei Normaldruck gearbeitet.

Die erfindungsgemäßen Verbindungen der Formel (I) zeigen eine überraschend gute und breite Wirkung gegen folgende Nematoden und Cestoden:

1. Hakenwürmer (z.B. Uncinaria stenocephala, Acylostoma caninum, Bunostomum trigonocephalum)
2. Trichostrongyliden (z.B. Nippostrongylus muris, Haemonchus contortus, Trichostrongylus colubriformis, Ostertagia circumcincta)
3. Strongyliden (z.B. Oesophagostomum columbianum)
4. Rhabdtiden (z.B. Strongyloides ratti)
5. Spulwürmer (z.B. Ascaris suum, Toxocara canis, Tocascaris leonina),
6. Madenwürmer (z.B. Aspiculuris tetraptera)
7. Heterakiden (z.B. Heterakis spumosa)
8. Peitschenwürmer (z.B. Trichuris muris)
9. Filarien (z.B. Litomosoides carinii, Dipetalonema witei)
10. Cestoden (z.B. Hymenolepis nana, Taenia pisiformis, Echinococcus multilocularis)
11. Trematoden (z.B. Fasciola hepatica)

Die Wirkung wurde im Tierversuch nach oraler und parenteraler Applikation bei stark mit Parasiten befallenen Versuchstieren geprüft. Die angewendeten Dosierungen wurden sehr gut von den Versuchstieren vertragen.

Die neuen Wirkstoffe können als Anthelmintika sowohl in der Human- als auch in der Veterinärmedizin verwendet werden.

Die neuen Wirkstoffe können in bekannter Weise in die üblichen Formulierungen übergeführt werden.

Die neuen Verbindungen können entweder als solche oder aber in Kombination mit pharmazeutisch annehmbaren Trägern zur Anwendung gelangen. Als Darreichungsformen in Kombination mit verschiedenen inerten Trägern kommen Tabletten, Kapseln, Granulate, wäßrige Suspensionen, injizierbare Lösungen, Emulsionen und Suspensionen, Elikiere, Sirup, Pasten und dergleichen in Betracht. Derartige Träger umfassen feste Verdünnungsmittel oder Füllstoffe, ein steriles, wäßriges Medium sowie verschiedene nicht toxische organische Lösungsmittel und dergleichen. Selbstverständlich können die für eine orale Verabreichung in Betracht kommenden Tabletten und dergleichen mit Süßstoffzusatz und ähnlichem versehen werden. Die therapeutisch wirksame Verbindung soll im vorgenannten Fall in einer Konzentration von etwa 0,5 bis 90 Gew.-% der Gesamtmischung vorhanden sein, d.h. in Mengen, die ausreichend sind, um den obengenannten Dosierungsspielraum zu erreichen.

Die Formulierungen werden in bekannter Weise hergestellt, z.B. durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet können.

Als Hilfsstoffe seien beispielhaft aufgeführt: Wasser, nicht toxische organische Lösungsmittel, wie Paraffine (z.B. Erdölfraktionen), pflanzliche Öle (z.B. Erdnuß-/Sesamöl), Alkohole (z.B. Äthylalkohol, Glycerin), Glykole (z.B. Propylenglykol, Polyäthylenglykol) und Wasser; feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker); Emulgiermittel, wie nicht ionogene und anionische Emulgatoren (z.B. Polyoxyäthylen-Fettsäure-Ester, Polyoxyäthylen-Fettalkohol-Äther, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Im Falle der oralen Anwendung können Tabletten selbstverständlich außer den genannten Trägerstoffen auch Zusätze, wie Natriumcitrat, Calciumcarbonat und Dicalciumphosphat, zusammen mit verschiedenen Zuschlagstoffen, wie Stärke, vorzugsweise Kartoffelstärke, Gelatine und dergleichen, enthalten. Weiterhin können Gleitmittel, wie Magnesiumstearat, Natriumlaurylsulfat und Talkum, zum Tablettieren mitverwendet werden.

Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Wirkstoffe können in Kapseln, Tabletten, Pastillen, Dragees, Ampullen usw. auch in Form von Dosierungseinheiten enthalten sein, wobei jede Dosierungseinheit so angepaßt ist, daß sie eine einzelne Dosis des aktiven Bestandteils liefert.

Die neuen Verbindungen können in den Formulierungen auch in Mischungen mit anderen bekannten Wirkstoffen vorliegen.

Die neuen Wirkstoffe können in üblicher Weise angewenden werden. Die Applikation erfolgt vorzugsweise oral, eine parenterale, insbesondere subkutane, aber auch eine dermale Applikation sind jedoch ebenfalls möglich.

Im allgemeinen hat es sich als vorteilhaft erwiesen, Mengen von etwa 0,1 bis etwa 50 mg der neuen Verbindungen je kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen. Für die Applikation in der Human- und Veterinärmedizin ist der gleiche Dosierungsspielraum vorgesehen. Sinngemäß gelten auch die weiteren obigen Ausführungen.

Die anthelmintische Wirksamkeit der erfindungsgemäßen Wirkstoffe sei anhand der folgenden Anwendungsbeispiele näher erläutert.

### Beispiel A

*Hakenwurm-Test / Hund*

Experimentell mit *Ancylostoma caninum* infizierte Hunde wurden nach Ablauf der Praepatenzzeit der Parasiten behandelt.

Die Wirkstoffmenge wurde als reiner Wirkstoff oder 10%ig in Milchsäure gelöst in Gelatinekapseln *oral* appliziert.

Der Wirkungsgrad wird dadurch bestimmt, daß man die nach der Behandlung abgetriebenen und im Versuchstier verbliebenen Würmer nach Sektion zählt und den Prozentsatz der abgetriebenen Würmer errechnet.

Beim oben beschriebenen Test zeigten u.a. die Wirkstoffe aus den Beispielen 1, 13, 18, 25 eine gute Wirksamkeit.

### Beispiel B

*Hakenwurm-Test / Hund*

Experimentell mit *Uncinaria stenocephala* infizierte Hunde wurden nach Ablauf der Praepatenzzeit der Parasiten behandelt.

Die Wirkstoffmenge wurde als reiner Wirkstoff oder 10%ig in Milchsäure gelöst in Gelatinekapseln *oral* appliziert.

Der Wirkungsgrad wird dadurch bestimmt, daß man die nach der Behandlung abgetriebenen und im Versuchstier verbliebenen Würmer nach Sektion zählt und den Prozentsatz der abgetriebenen Würmer errechnet.

Beim oben beschriebenen Test zeigten u.a. die Wirkstoffe aus den Beispielen 1, 13, 18, 25 eine gute Wirksamkeit.

### Beispiel C

*Spulwurm-Test / Hund*

Natürlich mit *Toxocara canis* bzw. *Toxoscaris leonina* infizierte Hunde wurden *oral* behandelt.

Die Wirkstoffmenge wurde als reiner Wirkstoff oder 10% in Milchsäure gelöst in Gelatinekapseln oral appliziert.

Der Wirkungsgrad wird dadurch bestimmt, daß man die nach der Behandlung abgetriebenen und in den Versuchstieren verbliebenen Würmer nach Sektion zählt und den Prozentsatz der abgetriebenen Würmer errechnet.

Beim oben beschriebenen Test zeigten u.a. die Wirkstoffe aus den Beispielen 1, 13, 18, 25 eine gute Wirksamkeit.

Beispiel 1

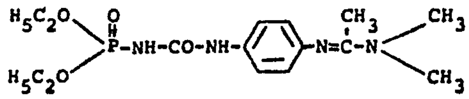

Zu einer Lösung von 59,3 g N-(4-Aminophenyl)-N',N'-dimethylacetamidin in 300 ml Tetrahydrofuran tropft man bei Raumtemperatur 71,9 g Diäthylphosphono-isocyanat, rührt 3 Stdn. bei 20°C und saugt das Reaktionsprodukt, N-[4-(3-Diäthylphosphonoureido)-phenyl]-N',N'-dimethylacetamidin, ab. Ausbeute 95,5 g=80% d.Th., Fp. 162—164°C (Zers.), Hydrochlorid: Fp. 180—181°C (Zers.);

In entsprechender Arbeitsweise erhält man:

mit Dimethylphosphono-isocyanat das N-[4-(3-Dimethylphosphonoureido)-phenyl]-N',N'-dimethylacetamidin, (Beispiel 2)

mit Dipropylphosphono-isocyanat das N-[4-(3-Dipropylphosphonoureido)-phenyl]-N',N'-dimethylacetamidin, (Beispiel 3)

mit Diisopropylphosphono-isocyanat das N-[4-3-(Diisopropylphosphonoureido)-phenyl]-N',N'-dimethylacetamidin, (Beispiel 4)

mit Dibutylphosphono-isocyanat das N-[4-(3-Dibutylphosphonoureido)-phenyl]-N',N'-dimethylacetamidin, (Beispiel 5)

mit Diisobutylphsophono-isocyanat das N-[4-(3-Diisobutylphosphonoureido)-phenyl]-N',N'-dimethylacetamidin, (Beispiel 6)

mit Di-sec-butylphosphono-isocyanat das N-[4-(3-Di-sec-butylphosphonoureido)-phenyl]-N',N'-dimethylacetamidin, (Beispiel 7)

mit Methyläthylphosphono-isocyanat das N-[4-(3-Methyläthylphosphonoureido)-phenyl]-N',N'-dimethylacetamidin, (Beispiel 8)

mit Äthyl-isopropylphosphono-isocyanat das N-[4-(3-Äthylisopropylphosphonoureido)-phenyl]-N',N'-dimethylacetamidin, (Beispiel 9).

Ferner sind nach dem in Beispiel 1 beschriebenen Verfahren unter anderem folgende erfindungsgemäße Verbindungen zugänglich.

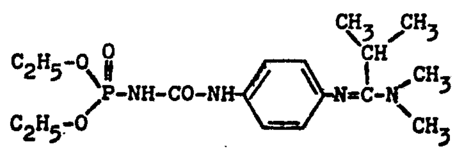

(Beispiel 10)

Fp: 182°C (Zers.)

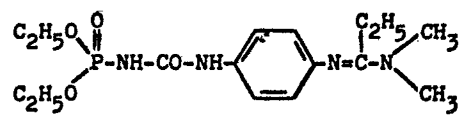

(Beispiel 11)

Fp: 163°C

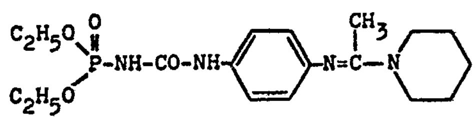

(Beispiel 12)

Fp: 184°C

Beispiel 13

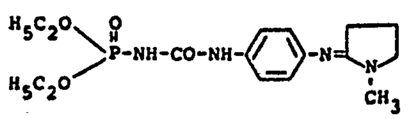

Nach der in Beispiel 1 beschriebenen Arbeitsweise erhält man aus Diäthylphosphono-isocyanat und 2-(4-Aminophenyl)imino-1-methyl-pyrrolidin das 2-[4-(3-Diäthylphosphonoureido)-phenyl]-1-methyl-pyrrolidin in einer Ausbeute von 95%, F. 159°C (Zers.).

In entsprechender Arbeitsweise erhält man:

mit Dipropylphosphono-isocyanat und 2-(4-Aminophenyl)-imino-1-methyl-pyrrolidin das 2-[4-(3-Dipropylphosphonoureido)phenyl]-1-methyl-pyrrolidin, (Beispiel 14)

mit Methyl-äthylphosphono-isocyanat und 2-(4-Aminophenyl)imino-1-methyl-pyrrolidin das 2-[4-(3-Methyl-äthylphosphonoureido)-phenyl]-1-methyl-pyrrolidin, (Beispiel 15)

mit Äthyl-propylphosphono-isocyanat und 2-(4-Aminophenyl)-imino-1-methyl-pyrrolidin das 2-[4-(3-Äthyl-propylphosphonoureido)-phenyl]-1-methyl-pyrrolidin, (Beispiel 16)

mit Diisopropylphosphono-isocyanat und 2-(4-Aminophenyl)-imino-1-methyl-pyrrolidin das 2-[4-(4-Diisopropylphosphonoureido)-phenyl]-1-methyl-pyrrolidin, (Beispiel 17).

Beispiel 18

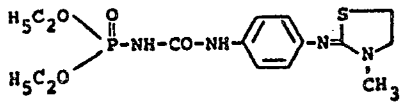

Nach der in Beispiel 1 beschriebenen Arbeitsweise erhält man aus Diäthylphosphono-isocyanat und 2-(4-Aminophenyl)-imino-3-methyl-1,3-thiazolidin das 2-[4-(3-Diäthylphosphonoureido)-phenyl]-3-methyl-1,3-thiazolidin in einer Ausbeute von 92%, Fp. 177—178°C.

In entsprechender Arbeitsweise erhält man:

mit Dimethylphosphono-isocyanat und 2-(4-Aminophenyl)-imino-3-methyl-1,3-thiazolidin das 2-[4-(3-Dimethylphosphonoureido)-phenyl]-3-methyl-1,3-thiazolidin, (Beispiel 19)

mit Dipropylphosphono-isocyanat und 2-(4-Aminophenyl)-imino-3-methyl-1,3-thiazolidin das 2-[4-(3-Dipropylphosphonoureido)-phenyl]-3-methyl-1,3-thiazolidin, (Beispiel 20)

mit Ethyl-propylphosphono-isocyanat und 2-(4-Aminophenyl-imino-3-methyl-1,3-thiazolidin das 2-[4-(3-Ethyl-propylphosphonoureido)-phenyl]-3-methyl-1,3-thiazolidin, (Beispiel 21)

mit Diisopropylphosphono-isocyanat und 2-(4-Aminophenyl)-imino-3-methyl-1,3-thiazolidin das 2-[4-(3-Diisopropylphosphono-ureido)-phenyl]-3-methyl-1,3-thiazolidin, (Beispiel 22).

Ferner sind nach dem in Beispiel 18 beschriebenen Verfahren unter anderem folgende erfindungsgemäße Verbindungen zugänglich:

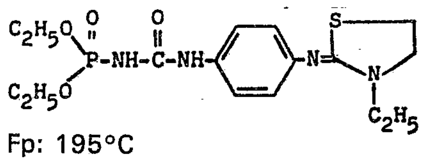

(Beispiel 23)

Fp: 195°C

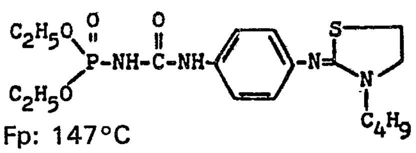

(Beispiel 24)

Fp: 147°C

Beispiel 25

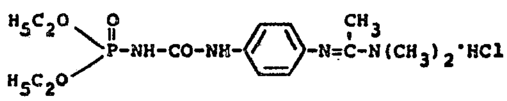

Zu 10,7 g (0,05 Mol) N-(4-Aminophenyl)-N',N'-dimethylacetamidin-hydrochlorid, gelöst in 400 ml Äthanol und 100 ml Tetrahydrofuran, tropft man bei 0°C 13,4 g (0,075 Mol) Diäthylphosphono-isocyanat, rührt eine Stunde bei 20°, fügt nochmals 13,4 g Diäthylphosphono-isocyanat hinzu und rührt 5 Stunden. Nach dem Einengen im Vakuum, Verreiben mit Essigester und Umlösen aus Isopropanol erhält man 6,7 g Hydrochlorid, Fp.: 180—181°C (Zers.).

**Patentansprüche**

1. Phosphonylureidobenzol-Derivate gekennzeichnet durch die Formel

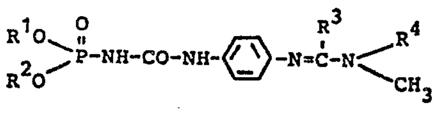

in welcher

$R^1$ und $R^2$ gleich oder verschieden sind und für Alkyl ($C_1$—$C_4$) stehen

$R^3$ und $R^4$ gleich oder verschieden sind und für Alkyl ($C_1$—$C_4$) stehen oder

$R^3$ und $R^4$ zusammen für eine Trimethylen- oder Tetramethylengruppe stehen oder

$R^3$ und $R^4$ zusammen mit dem durch sie eingeschlossenen Kohlenstoff- und Stickstoffatom ein Thiazolidin-Ringsystem bilden
sowie ihre physiologisch verträglichen Säureadditionssalze.

2. Phosphonylureidobenzol-Derivat nach Anspruch 1, gekennzeichnet durch die Formel

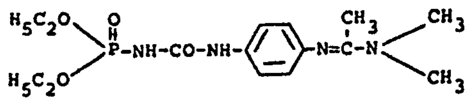

bzw. das entsprechende Säureadditionssalz.

3. Phosphonylureidobenzol-Derivat nach Anspruch 1, gekennzeichnet durch die Formel

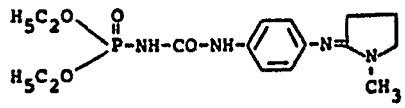

bzw. das entsprechende Säureadditionssalz.

4. Phosphonylureidobenzol-Derivat nach Anspruch 1, gekennzeichnet durch die Formel

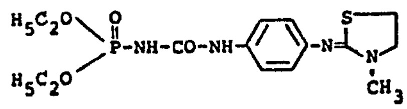

bzw. das entsprechende Säureadditionssalz.

5. Verfahren zur Herstellung von Phosphonylureidobenzol-Derivaten der allgemeinen Formel

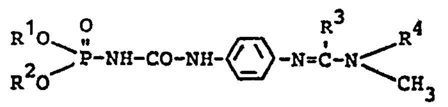

in welcher
$R^1$, $R^2$, $R^3$ und $R^4$ die in Anspruch 1 angegebene Bedeutung besitzen,
sowie ihre physiologisch verträglichen Säureadditionssalze, dadurch gekennzeichnet, daß man Aminophenylamidine der allgemeinen Formel

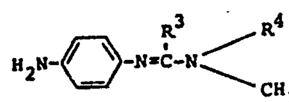

(II)

in welcher
$R^3$ und $R^4$ die in Anspruch 1 angegebene Bedeutung besitzen,
oder deren Salze mit Phosphonylisocyanaten der allgemeinen Formel

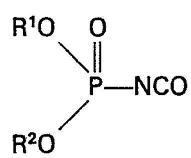

(III)

in welcher
$R^1$ und $R^2$ die in Anspruch 1 angegebene Bedeutung besitzen,
gegebenenfalls in Anwesenheit eines Lösungsmittels umsetzt, das Reaktionsprodukt gegebenenfalls isoliert und gegebenenfalls durch Zusatz von Säure in physiologisch verträgliche Salze überführt.

6. Arzneimittel, gekennzeichnet durch einen Gehalt an mindestens einem Phosphonylureidobenzol-Derivat gemäß Ansprüchen 1 bis 4.

7. Verfahren zur Herstellung von anthelmintischen Mitteln, dadurch gekennzeichnet, daß man Phosphonylureidobenzol-Derivate gemäß Ansprüchen 1 bis 4 mit inerten, nicht-toxischen, pharmazeutisch geeigneten Trägerstoffen vermischt.

**Claims**

1. Phosphonylureidobenzene derivates characterised by the formula

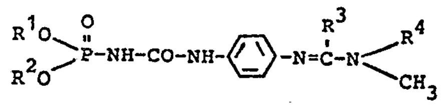

in which

$R^1$ und $R^2$ die in Anspruch 1 angegebene Bedeutung besitzen.

$R^3$ and $R^4$ are identical or different and represent alkyl ($C_1$—$C_4$) or

$R^3$ and $R^4$ together represent a trimethylene or tetramethylene group or

$R^3$ and $R^4$, together with the carbon atom and nitrogen atom between them, form a thiazolidine ring system,

and their physiologically acceptable acid addition salts.

2. A phosphonylureidobenzene derivate according to Claim 1, characterised by the formula

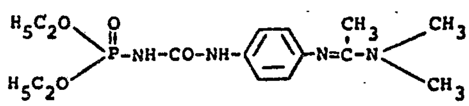

and the corresponding acid addition salt.

3. A phosphonylureidobenzene derivate according to Claim 1, characterised by the formula

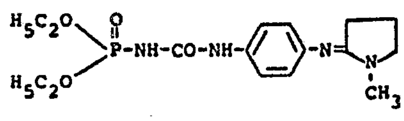

and the corresponding acid addition salt.

4. A phosphonylureidobenzene derivate according to Claim 1, characterised by the formula

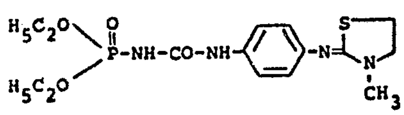

and the corresponding acid addition salt.

5. Process for the preparation of phosphonylureidobenzene derivatives of the general formula

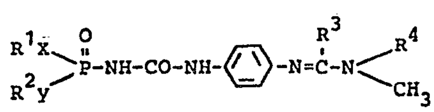

in which

$R^1$, $R^2$, $R^3$ and $R^4$ have the meaning indicated in Claim 1,
and their physiologically acceptable acid addition salts,
characterised in that aminophenylamidines of the general formula

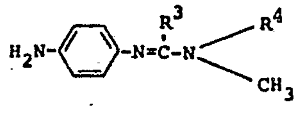

(II)

in which

$R^3$ and $R^4$ have the meaning indicated in Claim 1, or salts thereof, are reacted with phosphonyl isocyanates of the general formula

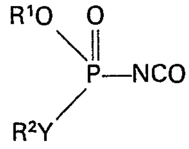

(III)

in which

$R^1$ and $R^2$ have the meaning indicated in Claim 1, optionally in the presence of a solvent, the reaction product is optionally isolated and optionally converted into physiologically acceptable salts by adding acid.

6. Medicaments, characterised in that they contain at least one phosphonylureidobenzene derivate according to Claims 1 to 4.

7. Process for the preparation of anthelmintic agents, characterised in that phosphonylureidobenzene derivatives according to Claims 1 to 4 are mixed with inert, non-toxic, pharmaceutically suitable excipients.

**Revendications**

1. Dérivés du phosphonyluréidobenzène, caractérisés en ce qu'ils répondent à la formule

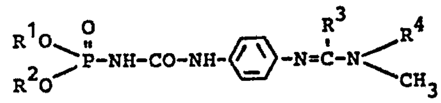

dans laquelle

$R^1$ et $R^2$, identiques ou différents, représentent des groupes alkyle en $C_1$—$C_4$,

$R^3$ et $R^4$, identiques ou différents, représentent des groupes alkyle en $C_1$—$C_4$, ou bien

$R^3$ et $R^4$ forment ensemble un groupe triméthylène ou tétraméthylène, ou bien

$R^3$ et $R^4$ forment ensemble et avec l'atome de carbone et l'atome d'azote qui les séparent, un système cyclique thiazolidine,

et leurs sels formés par addition avec des acides acceptables pour l'usage pharmaceutique.

2. Dérivé du phosphonyluréidobenzène selon la revendication 1, caractérisé en ce qu'il répond à la formule

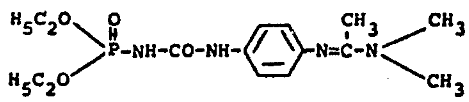

et ses sels formés par addition avec des acides.

3. Dérivé du phosphonyluréidobenzène selon la revendication 1, caractérisé en ce qu'il répond à la formule

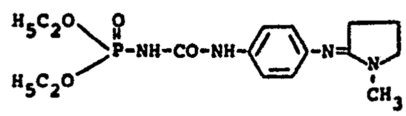

et ses sels formés par addition avec des acides.

4. Dérivé du phosphonyluréidobenzène selon la revendication 1, caractérisé en ce qu'il répond à la formule

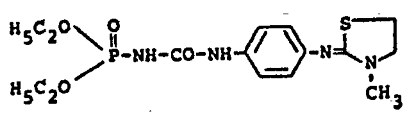

et ses sels formés par addition avec des acides.

5. Procédé de préparation de dérivés de phosphonyluréidobenzène répondant à la formule générale

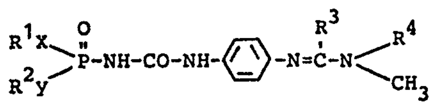

dans laquelle

$R^1$, $R^2$, $R^3$ et $R^4$ ont les significations indiquées dans la revendication 1, et de leurs sels formés par addition avec des acides acceptables pour l'usage pharmaceutique, ce procédé se caractérisant en ce que l'on fait réagir des aminophénylamidines répondant à la formule générale

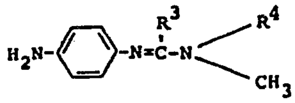

(II)

dans laquelle

$R^3$ et $R^4$ ont les significations indiquées dans la revendication 1, ou leurs sels, avec des phosphonylisocyanates répondant à la formule générale

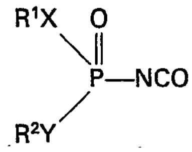

(III)

dans laquelle

$R^1$ et $R^2$ ont les significations indiquées dans la revendication 1, le cas échéant en présence d'un solvant, on isole éventuellement le produit de réaction et le cas échéant on le convertit par addition d'un acide en un sel acceptable pour l'usage pharmaceutique.

6. Médicaments caractérisés en ce qu'ils contiennent au moins un dérivé du phosphonyluréidobenzène selon les revendications 1 à 4.

7. Procédé pour la préparation de produits anti-helminthiques, caractérisé en ce que l'on mélange des dérivés du phosphonyluréidobenzène selon les revendications 1 à 4 avec des véhicules inertes non toxiques appropriés à l'usage pharmaceutique.